# EUROPEAN PATENT APPLICATION

(11) **EP 0 710 481 A1**
(43) Date of publication of application: **08.05.1996**
(21) Application number: 94203188.1
(22) Date of filing: 02.11.1994
(51) Int. Cl.: A61K 31/495

(54) **Use of flesinoxan for cognition enhancement**

(71) Applicant: DUPHAR INTERNATIONAL RESEARCH B.V, NL-1381 CP Weesp (NL)
(72) Inventor: Van Harten, Jaap c/o Octrooibureau Zoan B.V., NL-1380 AC Weesp (NL); Bradford, L. DiAnne c/o Octrooibureau Zoan B.V., NL-1380 AC Weesp (NL); De Koning, Paul c/o Octrooibureau Zoan B.V., NL-1380 AC Weesp (NL)
(74) Representative: Muis, Maarten

(57) **Abstract**

It has been found that (+)-4-fluoro-N-[2-[4-[5-(2-hydroxymethyl-1,4-benzodioxanyl)]-1-piperazinyl]ethyl]benzamide has cognition enhancing activity. This activity has been found by means of clinical trials.

## Description

The present invention relates to a new use of (+)-4-fluoro-N-[2-[4-[5-(2-hydroxymethyl-1,4-benzodioxanyl)]-1-piperazinyl]ethyl] benzamide (known as flesinoxan) and pharmacologically acceptable acid addition salts thereof.
It is known from EP 0138280 that flesinoxan shows blood pressure lowering activity. Further it is known from EP 0307061 that flesinoxan can be used for the treatment of generalised anxiety disorder and major depressive disorder.
Furthermore, it is described in WO 94/09780 that a group of piperazine derivatives of the formula 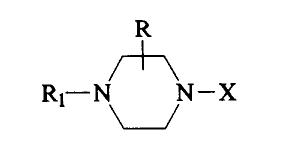
wherein R is hydrogen or lower alkyl, R₁ is an aryl or nitrogen containing heteroaryl radical, and X among others may represent a group -A-NR₆COR₇ (wherein A is an alkylene chain of 2-4 C-atoms, R₆ is a mono- or bicyclic heteroaryl radical, and R₇ among others may represent an aryl radical), is useful in the treatment of cognitive disorders.

It has now been found that flesinoxan in vivo has a remarkable cognition enhancing activity.
In a clinical trial which studied the pharmacodynamic interaction of flesinoxan with alcohol in young volunteers it was surprisingly found that cognitive performance was enhanced following repeated dosing with flesinoxan.
The effects found in this trial with young volunteers were so remarkable that a further study has been conducted to investigate the cognitive effects of flesinoxan in young (18-35 years), elderly (65-74 years) and very elderly (75 years or older) populations. This was a partly randomised, cross-over study involving 52 healthy volunteers, in three groups of 17, 18 and 17 respectively (12 on active medication during the whole study period and 5, 6 and 5 on placebo).

There were three study sessions: single-dose administration of flesinoxan or placebo with or without food on days 1 and 8, and multiple-dose administration of flesinoxan or placebo from day 10 until the morning dose of day 17.
Psychometric testing was conducted prior to dosing and at 1 and 3.5 hours following dosing on the single dosing day (day 1 or 8) when flesinoxan was taken in the fasting state, and also on day 17.
A selection of tasks from a Computerised Cognitive Assessment System of a specialized company was administered. All tasks are computer-controlled, the information being presented on high resolution monitors, and the response recorded via response modules containing two buttons, one marked "NO" and the other "YES". The tests were administered in the following order:
- Word Presentation: A list of 15 words is presented on the monitor at the rate of 1 every 2 seconds for the volunteer to remember.
- Immediate Word Recall: The volunteer is given 1 minute to recall as many of the words as possible.
- Picture Presentation: A series of 20 pictures is presented on the monitor at the rate of 1 every 3 seconds and the volunteer instructed to remember as many as possible.
- Simple Reaction Time: The volunteer is instructed to press the "YES" response button as quickly as possible every time the word "YES" is presented on the monitor. Thirty stimuli are presented with a varying inter-stimulus interval.
- Digit Vigilance Task: A target digit is randomly selected and constantly displayed to the right of the monitor screen. A series of digits is presented in the centre of the screen at the rate of 150 per minute and the volunteer is required to press the "YES" button as quickly as possible every time the digit in the series matches the target digit. There are 45 targets.
- Choice Reaction Time: Either the word "NO" or the word "YES" is presented on the monitor and the volunteer is instructed to press the corresponding button as quickly as possible. There are thirty trials for each of which the stimulus word is chosen randomly with equal probability and there is a varying inter-stimulus interval.
- Rapid Visual Information Processing Task: A series of digits is presented on the screen at the rate of 80 per minute. The volunteer has to detect targets consisting of consecutive sequences of either three odd digits or three even digits, and to report them by pressing the "YES" button as quickly as possible. There are 16 targets.
- Memory Scanning Task: A series of five digits is presented for the volunteer to hold in memory. For each of a subsequent series of 30 probes the volunteer must press the "YES" response button if it is one of the original digits and the "NO" response button if it is any other digit.
- Delayed Word Recall: The volunteer is again given 1 minute to recall as many of the words as possible.
- Delayed Word Recognition: The original words plus 15 distractor words are presented one at a time in a randomised order. For each word the volunteer must indicate whether or not he recognises it as being from the original list of words by pressing the "YES" or "NO" button as appropriate.
- Delayed Picture Recognition: The original pictures plus 20 distractor pictures are presented one at a time in a randomised order. The volunteer must indicate for each picture whether or not he believes it was from the series originally presented, by pressing the "YES" or "NO" button as appropriate.

The 52 volunteers each received, in a randomized order, two single doses of flesinoxan. HCl (0.5 mg free base) or placebo, one with food and the other without food, and subsequently flesinoxan. HCl (up to 1.0 mg free base b.i.d (12 per group), or placebo (5 or 6 per group) for one week. Subjects on flesinoxan received active drug during the whole study period. The two single-dose sessions were separated by a 7 days washout period.
The results of this study indicate a widespread set of improvements to cognitive function with flesinoxan. In the study protocol four outcome variables were defined as "primary". The other outcome variables were defined as secondary ("supportive"). For the primary variables, beneficial effects were seen for the accuracy of immediate word recall, the sensitivity of word recognition and the speed of digit vigilance. Each of these variables showed significant changes to occur as a result of repeated dosing. While the effects were seen generally, they were most pronounced and easily detected in the group of very-elderly volunteers. Improvements with flesinoxan were also seen on the following secondary tasks: simple reaction time, choice reaction time, rapid information processing and picture recognition sensitivity. The major effects seen on these variables were responses to the drug on the study days, and were seen across the age-groups. The most consistent effects were seen on the rapid information processing task and on picture recognition, all age-groups showing benefits after dosing with flesinoxan. There were only few isolated instances where flesinoxan was inferior to placebo.
The pattern of cognitive improvements found in this trial was similar to the one seen in the above mentioned previous trial in which flesinoxan was administered repeatedly to young volunteers in combination with alcohol.
Putting together the findings from these studies it appears that flesinoxan is capable of improving cognitive performance in young and elderly volunteers, these improvements becoming apparent after only a few days of dosing. Following repeated dosing there was evidence of improvements shortly after dosing, though where this was seen it was more likely to occur in elderly, suggesting an increased responsivity of this population to the drug. An alternative explanation would be that generally there is more room for improvement in the scores of elderly.
Based on the improvements found in the above described trial with healthy volunteers flesinoxan and its suitable acid addition salts are suitable for cognition enhancement in human beings.
According to the invention flesinoxan is active in obtaining cognition enhancement in oral dosages between 0.25 and 4.0 mg daily.
Flesinoxan can be formulated into compositions suitable for administration to human beings according to methods known per se.

## Claims

1. Use of (+)-4-fluoro-N-[2-[4-[5-(2-hydroxymethyl-1,4-benzodioxanyl)]-1-piperazinyl]ethyl] benzamide or a pharmacologically acceptable acid addition salts thereof for the preparation of a composition for cognition enhancement.

2. Use as claimed in claim 1, characterized in that a composition is prepared suitable for an oral daily dose of 0.25 - 4.0 mg of flesinoxan.

3. Use as claimed in claim 1 or 2, characterized in that flesinoxan. HCl is used as the active substance.

4. Method of enhancing cognition, characterized in that flesinoxan or a pharmacologically acceptable acid addition salt thereof is administered to a patient in need of such treatment.
